# EUROPEAN PATENT SPECIFICATION

(11) **EP 1 453 520 B1**
(45) Date of publication and mention of the grant of the patent: **06.09.2006**
(21) Application number: 01984842.3
(22) Date of filing: 14.12.2001
(51) Int. Cl.: A61K 31/565, A61K 31/715, A61K 31/726, A61K 31/737, A61K 31/375, A61K 31/401, A61K 33/04, A61K 33/34, A61K 33/26, A61P 5/30, A61P 5/24, A61K 31/569, A61K 31/567

(54) **COMPOSITION OF ESTROGEN AND OTHER HORMONES WITH ASCORBATE, LYSINE, PROLINE AND OTHER SUBSTANCES**
ZUSAMMENSETZUNG ENTHALTEND OESTROGEN ODER ANDERE HORMONE MIT ASCORBAT, LYSIN UND PROLIN ETC
COMPOSITION D'OESTROGENE ET D'AUTRES HORMONES COMBINEES A L'ASCORBATE, LA LYSINE, LA PROLINE ET A D'AUTRES SUBSTANCES

(43) Date of publication of application: 08.09.2004
(73) Proprietor: Rath, Matthias, 6422 PC Heerlen (NL)
(72) Inventor: RATH, Matthias, 6422 PC Heerlen (NL); NIEDZWIECKI, Aleksandra, San Jose, CA 95129 (US); NETKE, Shrirang, Shivaji Nagar, Nagpur (MS) 440010 (US); IVANOV, Vadim, Castro Valley, CA 94552 (US)
(74) Representative: Federhen, Ludwig
(86) International application number: PCT/EP2001/014726
(87) International publication number: WO 2003/051371

(56) References cited:
- EP-A- 0 688 785
- EP-A- 1 068 868
- DE-U- 29 916 231
- GB-A- 888 631
- US-A- 4 980 358
- HALL S L ET AL: "The relation of dietary vitamin C intake to bone mineral density: Results from the PEPI study." CALCIFIED TISSUE INTERNATIONAL, vol. 63, no. 3, 1998, pages 183-189, XP001088430 ISSN: 0171-967X

## Description

This invention concerns compositions of estrogen and related hormones in combination with ascorbate, lysine, proline and other substances effecting connective tissues, their ability and their use in contraception, hormonal replacement therapy during menopause, and for other use in medicine, physiology, pharmacology, pharmaceuticals and cosmetic application.

Estrogens and related hormones are widely used in medicine. Their primary applications are contraception and hormone replacement therapy during menopause and others.

Long-term use of estrogen and related hormones, however, has significant and frequent side - effects including cancer, particularly cancer of the breast, uterus and other malignancies in organs of the reproductive system, heart diseases and others which are related to extracellular matrix degeneration. According to some sources (Pschytembel, Klinisches Wörterbuch, 259 edition) the treatment with estrogens is considered to be contraindicated in cases of estrogen depending tumors, acute or chronic diseases of the liver, thrombo-embolie and hypertension. In case of thrombo-embolitic and ischemic diseases, increased hypertension estrogen treatment should be stopped immediately.

Estrogens and related hormones promote the spread of cancer and the exacerbation of other diseases by weakening the extracellular matrix. This effect of estrogen is mediated by the activation of connective tissue degrading enzymes including plasmin, collagenases and by other direct and indirect mechanism destabilising the connective tissue.

It is known from European Patent Application 00115643.9 published January 17, 2001 that pharmaceutical compositions comprising ascorbate or compositions comprising ascorbate and fibrinolyes inhibitors like lysine and proline combined with other substances like tocopherol, carotene, selenium, pro-vitamins and trace elements are suitable pharmaceutical compositions for the prevention and treatment of diseases of pathological states related to the degradation of the extracellular matrix including degenerative diseases particularly arteriosclerosis, cancers and other related diseases.

There is an obvious need to develop therapeutical strategies to minimize the side-effects of treatments with estrogen or otherwise beneficial hormone therapies.

It was supposed that when administering estrogen in combination with ascorbate, lysine, proline and other substances actions of each of theses compositions would cancel each other. Estrogen treatment leads to degradation of the extracellular matrix while known compositions of ascorbic acids, particularly in combination with lysine and proline, reduce or inhibit such degradations whereby estrogen treatment would become useless. However, surprisingly this is at least partly not the case because of the synergistic effect of such ascorbic acid compositions, particularly when combined with lysine and proline which on the one hand prevent or inhibit extracellular matrix degeneration, on the other hand however enhance collagen synthesis, particularly with ascorbic acid which creates and supports the extracellular matrix.

The compounds used in this invention for a combined use or therapeutic application with estrogens and related hormones are chosen to counteract the weakening of the connective tissue by those hormones. Ascorbate is known to stimulate the synthesis of collagen, elastin and other connective tissue macromolecules from fibroblast and related cells. The amino acids lysine and proline are the predominant amino acids required for the synthesis of connective tissue molecules. Iron and copper are trace elements that catalyze the production of these macromolecules.

During physiological conditions such as ovulation and pregnancy the degradation of connective tissue by estrogen is counteracted by a regulated system of natural mechanisms to prevent the degradation of collagen. Under pathological conditions caused by side-effects of beneficial hormone therapies the natural mechanism which is no more active during and after menopause can be replaced by the therapeutic use of the combinations according to this invention. They are useful to minimize or prevent side-effects of long-term hormone therapies including cancer and other severe health conditions while allowing the desired medical or therapeutic effect of estrogen and related hormones.

The different compounds claimed in this application can be used together in form of covalently bound compounds or as physical mixture or in any other combination.

## Claims

1. A pharmaceutical composition comprising estrogen, estradiol, ethinylestradiol, estriol, norethisterone or lynestrenol, and lysine, lysine salts, hydroxylysine or hydroxylysine salts, and ascorbic acid or ascorbate salts, and proline, proline salts, hydroxyproline or hydroxyproline salts.

2. The pharmaceutical composition of claim 1, further comprising selenium or selenium salts, copper or copper salts, iron or iron salts and other minerals or trace elements.

3. The pharmaceutical composition of claim 1 or 2, further comprising chondroitin sulfate, glycosaminoglycanes, proteoglycanes or glycoproteins of the extra cellular matrix.

4. Use of estrogen, estradiol, ethinylestradiol, estriol, norethisterone or lynestrenol, and lysine, lysine salts, hydroxylysine or hydroxylysine salts, and ascorbic acid or ascorbate salts, and proline, proline salts, hydroxyproline or hydroxyproline salts for the preparation of a pharmaceutical composition for control of fertility, contraception, for hormonal replacement therapy to alleviate symptoms related to menopause, to prevent osteoporosis during menopause, to prevent cardiovascular diseases during menopause, and to prevent other ailments during menopause.

5. The use of claim 4, wherein said pharmaceutical composition further comprises selenium or selenium salts, copper or copper salts, iron or iron salts and other minerals or trace elements.

6. The use of claim 4 or 5, wherein said pharmaceutical composition further comprises chondroitin sulfate, glycosaminoglycanes, proteoglycanes or glycoproteins of the extra cellular matrix.

7. The use of any one of claims 4 to 6, wherein said pharmaceutical composition is to be administered sublingually, intravenously, subcutaneously, intramuscularly, transdermally or parenterally.

## Patentansprüche

1. Eine pharmazeutische Zusammensetzung, die Estrogen, Estradiol, Ethinylestradiol, Estriol, Norethisteron oder Lynestrenol, und Lysin, Lysinsalze, Hydroxylysin oder Hydroxylysinsalze, und Ascorbinsäure oder Ascorbatsalze, und Prolin, Prolinsalze, Hydroxyprolin oder Hydroxyprolinsalze umfasst.

2. Die pharmazeutische Zusammensetzung nach Anspruch 1, die weiterhin Selen oder Selensalze, Kupfer oder Kupfersalze, Eisen oder Eisensalze und andere Mineralien oder Spurenelemente umfasst.

3. Die pharmazeutische Zusammensetzung nach Anspruch 1 oder 2, die weiterhin Chondroitinsulfate, Glycosaminoglycane, Proteoglycane oder Glycoproteine der extrazellulären Matrix umfasst.

4. Verwendung von Estrogen, Estradiol, Ethinylestradiol, Estriol, Norethisteron oder Lynestrenol, und Lysin, Lysinsalzen, Hydroxylysin oder Hydroxylysinsalzen, und Ascorbinsäure oder Ascorbatsalzen, und Prolin, Prolinsalzen, Hydroxyprolin oder Hydroxyprolinsalzen für die Herstellung einer pharmazeutischen Zusammensetzung für die Fruchtbarkeitskontrolle, Empfängnisverhütung, für eine Hormonersatztherapie zur Verringerung von Symptomen, die mit der Menopause verbunden sind, für eine Vorbeugung von Osteoporose während der Menopause, für eine Vorbeugung von Herz-Kreislauf-Krankheiten während der Menopause und für eine Vorbeugung von anderen Beschwerden während der Menopause.

5. Die Verwendung nach Anspruch 4, wobei die pharmazeutische Zusammensetzung weiterhin Selen oder Selensalze, Kupfer oder Kupfersalze, Eisen oder Eisensalze und andere Mineralien oder Spurenelemente umfasst.

6. Die Verwendung nach Anspruch 4 oder 5, wobei die pharmazeutische Zusammensetzung weiterhin Chondroitinsulfat, Glycosaminoglycane, Proteoglycane oder Glycoproteine der extrazellulären Matrix umfasst.

7. Die Verwendung nach einem der Ansprüche 4 bis 6, wobei die pharmazeutische Zusammensetzung sublingual, intravenös, subkutan, intramuskulär, transdermal oder parenteral zu verabreichen ist.

## Revendications

1. Composition pharmaceutique comprenant de l'estrogène, de l'estradiol, de l'éthinylestradiol, de l'estriol, de la noréthistérone ou du lynestrénol, et de la lysine, des sels de lysine, de l'hydroxylysine ou des sels d'hydroxylysine, et de l'acide ascorbique ou des sels d'ascorbate, et de la proline, des sels de proline, de l'hydroxyproline ou des sels d'hydroxyproline.

2. Composition pharmaceutique selon la revendication 1, qui comprend en outre du sélénium ou des sels de sélénium, du cuivre ou des sels de cuivre, du fer ou des sels de fer et autres minéraux ou éléments traces.

3. Composition pharmaceutique selon la revendication 1 ou 2, comprenant en outre du sulfate de chondroitine, des glycosaminoglycanes, des protéoglycanes ou des glycoprotéines de la matrice extracellulaire.

4. Utilisation d'estrogène, d'estradiol, d'éthinylestradiol, d'estriol, de noréthistérone ou de lynestrénol, et de lysine, de sels de lysine, d'hydroxylysine ou de sels d'hydroxylysine, et d'acide ascorbique ou de sels d'ascorbate, et de proline, de sels de proline, d'hydroxyproline ou de sels d'hydroxyproline pour la préparation d'une composition pharmaceutique utilisée dans le contrôle de la fertilité, la contraception, le traitement hormonal de remplacement pour soulager les symptômes liés à la ménopause, et pour empêcher l'ostéoporose, les maladies cardiovasculaires et d'autres affections pendant la ménopause.

5. Utilisation selon la revendication 4, dans laquelle ladite composition pharmaceutique comprend en outre du sélénium ou des sels de sélénium, du cuivre ou des sels de cuivre, du fer ou des sels de fer et autres minéraux ou éléments traces.

6. Utilisation selon la revendication 4 ou 5, dans laquelle ladite composition pharmaceutique comprend en outre du sulfate de chondroitine, des glycosaminoglycanes, des protéoglycanes ou des glycoprotéines de la matrice extracellulaire.

7. Utilisation selon l'une quelconque des revendications 4 à 6, dans laquelle ladite composition pharmaceutique doit être administrée par voie sublinguale, intraveineuse, sous-cutanée, intramusculaire, transdermique ou parentérale.
